**Europäisches Patentamt**

**European Patent Office**

**Office européen des brevets**

(19)

(11) Numéro de publication: **0 247 018**
**B1**

(12) **FASCICULE DE BREVET EUROPEEN**

(45) Date de publication du fascicule du brevet:
**31.01.90**

(51) Int. Cl.⁴: **A 01 G 7/00**

(21) Numéro de dépôt: **87870054.1**

(22) Date de dépôt: **22.04.87**

(54) Méthode et composition de lutte contre le phénomène de vitrification au cours de la micropropagation in vitro des plantes.

(30) Priorité: **23.04.86 BE 216580**

(43) Date de publication de la demande:
**25.11.87 Bulletin 87/48**

(45) Mention de la délivrance du brevet:
**31.01.90 Bulletin 90/5**

(84) Etats contractants désignés:
**AT CH DE ES FR GB GR IT LI NL SE**

(56) Documents cités:
**EP-A- 0 129 668**
**GB-A- 2 099 851**

(73) Titulaire: **Personnalité Juridique de la Station des Cultures Fruitières Maraîchères, 234 Chaussée de Charleroi, B-5800 Gembloux (BE)**

(72) Inventeur: **Boxus, Emile Philippe, rue de Liedekerke 22, B-1030 Bruxelles (BE)**
Inventeur: **Paques, Marc, 11 rue des Mésanges, B-5100 Jambes (BE)**

(74) Mandataire: **Kuborn, Jacques et al, Office Hanssens S.P.R.L. Square Marie-Louise, 40 (bte 19), B-1040 Bruxelles (BE)**

ACTORUM AG

## Description

L'invention se rapporte à la multiplication de plantes in vitro, et en particulier à la lutte contre des troubles de croissance des explants au cours de ce processus de multiplication.

Les plantes multipliées in vitro ont tendance à présenter des troubles de croissance qui peuvent perturber la production industrielle par micropropagation.

Les explants atteints portent des feuillers anormales plus ou moins enroulées, translucides, présentant souvent une couleur vert foncé; ils perdent leur capacité à proliférer, se nécrosent et meurent plus ou moins rapidement. Ce trouble est le plus souvent appelé «vitrification», «vitrescence» ou «hyperhydrie» et sera désigné ci-après par «vitrification». Il est d'autant plus fréquent ou intense que les plantes sont soumises à une multiplication plus intense.

L'emploi de milieu liquides augmente aussi les risques de vitrification.

NAVATEL (Fruits, vol. 37, n° 5, 331-336, 1982) a décrit le trouble comme suit:

«Ce symptôme qui apparaît généralement pendant la phase de multiplication a été signalé par de nombreux laboratoires. Les plants prennent un aspect «vitreux». Les feuilles, de couleur vert foncé et luisantes, se recourbent, deviennent cassantes et translucides. L'importance des dégâts semble augmenter avec le nombre de cultures sur le milieu prolifération, plus ou moins riche en cytokinine. Dans la majorité des cas, les plantes présentant ces symptômes se bloquent et ne sont pratiquement plus utilisables pour la suite de la multiplication. On observe quelquefois un certain rétablissement du végétal lorsque celui-ci est placé dans un milieu sans auxine et sans cytokinine. Ce type d'accident a été observé sur le GF 677 mais également sur différents porte-greffe du cerisier et du pommier.» «De nombreuses hypothèses ont été émises quant à l'origine de ces symptômes: milieu trop riche, toxicité de l'ion NF4, densité trop importante dans le bocal, production d'éthylène, mauvaise adaptation des bocaux de culture, toxicité par accumulation dans le végétal de certaines substances de croissance (cytokinine). Des travaux récents montrent que l'apparition de ce type de symptôme serait lié au potentiel hydrique du milieu (DEBERGH et al. 1981), alors que d'autres auteurs confirment le rôle prépondérant que jouerait l'ion NH4, peut-être en corrélation avec d'autres éléments, dans l'apparition de ce phénomène (BEAUCHESNE, 1981).»

«D'autres observations ont été faites par différents auteurs sur des techniques permettant sinon d'éliminer du moins de réduire la gravité de ces symptômes. ZUCCHERELLI (1979) montre que l'adjonction de pectine alimentaire au milieu de culture réduirait l'importance des plants «vitreux». DRUART (1980) signale que le passage du matériel végétal en chambre froide à plus ou moins 2°C pendant un mois environ limiterait l'apparition de tels symptômes.»

«Totefois, ces résultats ne permettent pas d'expliquer en totalité ce phénomène, probablement très complex du fait du nombre de facteurs pouvant interférer entre eux.»

L'agar est actuellement utilisé dans les milieux de culture, généralement en quantité de 7 à 12 g/l, pour les solidifier. Ceci facilite la mise en place des plantes et réduit dans une certaine mesure les risques de vitrification. Toutefois, le taux de multiplication est toujours plus élevé en milieu liquide qu'en milieu solide, et ce d'autant plus que la quantité d'agar est supérieure à environ 7 g/l.

Les solutions proposées n'ont apporté qu'une solution très limitée à la vitrification, les symptômes réapparaissant après 2 ou 3 mulriplications sur le milieu amélioré proposé.

Quant à la solution proposée par DEBERGH et al (Physiol. Plant. 59.270-276, 1981) d'augmenter la quantité d'agar du milieu solide, elle peut protéger les plants de la vitrification, mais entraîne une très forte réduction du taux de multiplication. L'effet de l'agar est dans ce cas simplement d'augmenter le potentiel matriciel du milieu, et est tout à fait différent de celui qu'il a dans l'invention, où l'on travaille en milieu liquide, c'est-à-dire avec un potentiel matriciel nul. De plus, l'augmentation de la quantité d'agar augmente le caractère solide du milieu de culture, et réduit d'autant le taux de multiplication. La relative «protection» contre la vitrification est donc obtenue en fait par passage à des conditions de multiplication moins intense.

Un but de l'invention est donc d'apporter une solution à la lutte contre le trouble de vitrification, sans perte du taux de multiplication.

Une caractéristique de l'invention concerne une méthode de lutte contre les troubles de croissance, désignés par vitrification, vitrescence ou hyperhydrie, dans la micropropagation de plantes multipliées in vitro, qui consiste à incorporer dans un milieu de culture une quantité efficace d'un hydrolysat d'agar.

Selon une autre caractéristique de l'invention, la quantité d'hydrolysat d'agar dans le milieu de culture est d'au moins 0,1 g/l (calculée sur l'agar avant hydrolyse) de milieu nutritif.

Une autre caractéristique de l'invention concerne une composition d'un milieu de culture pour la lutte contre la vitrification de plantes multipliées par micropropagation in vitro, qui contient un hydrolysat d'agar à titre de principe actif.

L'invention concerne encore un procédé de préparation d'une composition d'un milieu de culture comme ci-dessus, qui consiste à hydrolyser l'agar en milieu acide, à une température située entre 20 et 200°C, pendant 10 minutes à 7 jours. De préférence, l'hydrolyse est effectuée à une température située entre 100 et 120°C, pendant 30 à 60 minutes, à pH 3-4.

Enfin, selon l'invention, le milieu acide peut être de l'eau acidifiée ou le milieu de culture acidifié, selon que l'on souhaite obtenir l'hydrolysat d'agar pur, à ajouter à un milieu de culture, ou préparer directement le milieu de culture contenant l'hydrolysat d'agar en quantité voulue.

La solution proposée par l'invention consiste donc à introduire dans le milieu nutritif un hydrolysat d'agar en quantité efficace. D'après les essais menés par les inventeurs, la quantité d'hydrolysat d'agar n'est pas critique, pour autant qu'elle soit supé-

rieure à 0,1 g/l (calculée sur l'agar avant hydrolyse) de milieu nutritif, seuil en dessous duquel aucune activité n'a été observée.

L'invention s'applique à l'ensemble des espèces végétales, et plus particulièrement aux espèces ligneuses, dont notamment les Prunus et Malus parmi les fruitiers, pour lesquelles les inventeurs disposent d'une méthode d'induction de la vitrification, et sur lesquelles ont particulièrement portés les essais.

Elle semble efficace également pour les espèces non ligneuses, bien que les inventeurs ne disposent pas jusqu'à présent, pour ces espèces, d'une méthode d'induction de la vitrification permettant d'effectuer des essais comparatifs strictement reproductibles.

L'hydrolysat d'agar est liquide, et ne solidifie donc plus le milieu de culture, la multiplication se faisant dès lors en milieu liquide, soit dans les conditions les plus favorables du point de vue du taux de multiplication, mais offrant plus de risque d'apparition d'une vitrification des plantes.

L'agar est hydrolysé, soit seul dans de l'eau, soit avec le milieu de culture liquide.

Le milieu de culture utilisé par les inventeurs est le milieu proposé par DRUART (Colloque International sur la culture in vitro des essences forestières, pp 101-108, 1981, IUFRO Fontainebleau, France).

Le modèle d'introduction de la vitrification proposé par PAQUES (Arch. Int. Phys. et Bioch. 1984, 92, 20), qui fait appel à l'emploi d'un milieu liquide dans lequel sont plongées les bases des tiges des microplantes, sert de contrôle d'efficacité d'action de l'hydrolysat d'agar.

Une méthode de mise en œuvre de l'invention consiste à introduire l'agar dans le milieu de culture choisi, comme s'il s'agissait de préparer un milieu de culture solide classique, en quantité de 0,1 à 100 g/l.

L'ensemble est alors hydrolysé en milieu acide, pH 0-7, de préférence pH 0-4, mieux pH 3-4, à une température comprise entre 20-200°C, de préférence 50-150°C, mieux 75-120°C, pendant des temps variables selon la température et le pH, de 10 minutes à 7 jours. L'acide utilisé pour l'acidification n'est pas un paramètre de l'invention, et est généralement quelconque pour autant qu'il n'interfère pas avec le processus de multiplication. Ce peut être par exemple l'acide chlorhydrique, l'acide sulfurique, les acides phosphoriques , les acides carboxyliques et analogues.

Un mode de réalisation préféré de l'hydrolyse selon l'invention consiste à effectuer l'hydrolyse à pH 3-4, à une température de 75 à 120°C. L'hydrolyse dure alors de 30 à 40 minutes.

L'hydrolysat obtenu, ou le surnageant issu de la centrifugation, est alors utilisé comme milieu de culture. Des essais ont en effet montré que le culot de la centrifugation était inerte, et ne jouait aucun rôle dans le processus de lutte contre la vitrification selon l'invention. Il peut donc être éliminé si on le souhaite, pour une question de commodité.

L'hydrolyse du milieu de culture n'est cependant pas utile selon l'invention et on peut également préparer un hydrolysat d'agar «pur», c'est-à-dire sans milieu de culture. On peut ainsi préparer un hydrolysat concentré, par exemple en ajoutant à l'agar la quantité d'eau strictement nécessaire pour réaliser l'hydrolyse. Cet hydrolisat concentré peut alors être ajouté tel quel au milieu de culture choisi, pour protéger les plantes contre le risque de vitrification.

Dans la présente description, les quantités d'hydrolysat d'agar sont exprimées en gramme/litre de milieu de culture, et se rapportent au poids d'agar avant hydrolyse.

Comme on l'a indiqué plus haut, les inventeurs ont trouvé que la quantité d'hydrolysat d'agar n'était pas un paramètre significatif de l'invention, pour autant qu'elle soit égale ou supérieure à la quantité efficace, soit de l'ordre de 0,1 g/l. Des quantités préférées sont de 1 à 100 g/l, mieux de 7 à 12 g/l, soit des quantités équivalentes à celles utilisées actuellement pour solidifier les milieux de culture.

De même, l'origine de l'agar ne semble pas avoir d'influence sur les propriétés de lutte contre la vitrification. L'agar utilisé par les inventeurs est l'agar de qualité «Bacto» de DIFCO.

APPLICATION DU PROCEDE

Des microboutures du sujet porte-greffe de pommier M 26 sont cultivées soit sur un milieu de prolifération liquide contenant un hydrolysat d'agar, soit sur le même milieu sans cet hydrolysat.

Les milieux de culture sont préparés comme suit:

— milieu avec hydrolysat d'agar: la solution est constituée des macroéléments, des microéléments, des vitamines, des substances de croissance et de sucrose aux concentrations proposées par Druart (1981). L'agar «Bacto» de DIFCO est ajouté à la concentration de 7 g/l;

— milieu sans hydrolysat d'agar: ce milieu contient tous les éléments précités excepté l'agar.

Le pH des milieux réalisés est ajusté à 3,5 avec l'acide chlorhydrique, à la température du laboratoire. Les solutions sont alors soit portées à ébullition, distribuées dans les flacons de culture puis autoclavées 40 minutes à 110°C, soit autoclavées directement dans les conditions précitées puis distribuées stérilement dans les flacons de culture.

Les milieux ainsi obtenus sont liquides, toutefois, le milieu contenant l'agar hydrolysé flocule légèrement.

Le milieu sans agar (milieu A) est utilisé tel quel; le milieu contenant l'hydrolysat d'agar étant soit utilisé tel quel (milieu $B_1$), soit centrifugé à 300 g pendant 10 minutes. Le surnageant obtenu constitue le milieu de culture $B_2$. Le culot remis en suspension dans le milieu sans agar est le milieu $B_3$.

Repiquage des explants:

20 microboutures de M 26, de 2 cm de long sont repiquées sur les quatre milieux nutritifs décrits ci-dessus. Les observations réalisées, après trente jours de culture figurent dans le tableau suivant:

| Observations | Milieux | | | |
|---|---|---|---|---|
| | A | $B_1$ | $B_2$ | $B_3$ |
| Taux de Vitrification | 20/20 | 0/20 | 0/20 | 19/20 |
| Nombre de bourgeons/plante | 4-7 | 5-7 | 6-7 | 5-6 |

Bien entendu, l'invention n'est pas limitée aux modes de réalisation décrits, qui n'ont été choisis qu'à titre d'exemple.

**Revendications**

1. Méthode de lutte contre les troubles de croissance, désignés par vitrification, vitrescence ou hyperhydrie, dans la micropropagation de plantes multipliées in vitro, caractérisée en ce qu'elle consiste à incorporer dans un milieu de culture une quantité efficace d'un hydrolysat d'agar.

2. Méthode selon la revendication 1, caractérisé en ce que l'hydrolysat d'agar est incorporé en quantité d'au moins 0,1 g/l (calculée sur l'agar avant hydrolyse) de milieu de culture.

3. Composition d'un milieu de culture pour la lutte contre la vitrification de plantes multipliées par micropropagation in vitro, caractérisé en ce qu'elle contient un hydrolysat d'agar à titre de principe actif.

4. Procédé de préparation d'une composition d'un milieu de culture selon la revendication 3, caractérisé en ce qu'il consiste à hydrolyser l'agar en milieu acide, à une température située entre 20 et 200°C, pendant 10 minutes à 7 jours.

5. Procédé selon la revendication 4, caractérisé en ce que l'hydrolyse est effectuée à une température située entre 100 et 120°C, pendant 30 à 60 minutes, à pH 3-4.

6. Procédé selon la revendication 4, caractérisé en ce que le milieu acide est de l'eau acidifiée.

7. Procédé selon la revendication 4, caractérisé en ce que le milieu acide est le milieu de culture acidifié.

**Patentansprüche**

1. Verfahren zur Bekämpfung von Wachstumsproblemen, erkennbar als Vitrifikation, Vitreszenz oder Hyperhydration bei der Mikrovermehrung von in-vitro vermehrten Pflanzen, dadurch gekennzeichnet, daß eine wirksame Menge von hydrolisiertem Agar in die Kultur eingeführt wird.

2. Verfahren nach Anspruch 1, dadurch gekennzeichnet, daß das hydrolisierte Agar der Kultur in einem Mengenverhältnis von mindestens 0,1 g/l (berechnet nach dem Agar vor der Hydrolyse) zugesetzt wird.

3. Zusammensetzung eines Kulturmediums zur Bekämpfung der Vitrifikation von in-vitro vermehrten Pflanzen, dadurch gekennzeichnet, daß sie hydrolysiertes Agar als Aktivbestandteil enthält.

4. Verfahren zum Bereiten einer Zusammensetzung nach Anspruch 3, dadurch ghekennzeichnet, daß dieses aus einer Hydrolyse von Agar in saurem Medium bei einer Temperatur zwischen 20-200°C für 10 Minuten bis 7 Tagen besteht.

5. Verfahren nach Anspruch 4, dadurch gekennzeichnet, daß die Hydrolyse bei einer Temperatur zwischen 100 und 120°C für 30 bis 60 Minuten bei einem pH-Wert von 3-4 durchgeführt wird.

6. Verfahren nach Anspruch 4, dadurch gekennzeichnet, daß das saure Medium ein angesäuertes Wasser ist.

7. Verfahren nach Anspruch 4, dadurch gekennzeichnet, daß die saure Umgebung das angesäuerte Kulturmedium ist.

**Claims**

1. Process for combating growth disturbances, called vitrification, vitrescence or hyperhydria, in the in vitro micropropagation of plants, characterized in that it comprises incorporating into a culture medium an effective amount of agar hydrolysate.

2. Process according to claim 1, characterized in that the agar hydrolysate is included in an amount of at least 0,1 g/l (calculated on the basis of agar before hydrolysis) relative to the culture medium.

3. Composition of a culture medium for combating vitrification in the in vitro micropropagation of plants, characterized in that it contains agar hydrolysate as the active ingredient.

4. Process for preparing a composition of a culture medium according to claim 3, characterized in that it comprises hydrolysing agar in an acid medium, at a temperature of between 20 and 200°C, for 10 minutes to 7 days.

5. Process according to claim 4, characterized in that hydrolysis is made at a temperature of between 100 and 120°C, for 30 to 60 minutes, at a pH of 3-4.

6. Process according to claim 4, characterized in that the acid medium is acidified water.

7. Process according to claim 4, characterized in that the acid medium is the acidified culture medium.